# EUROPEAN PATENT SPECIFICATION

(11) **EP 3 991 721 B1**
(45) Date of publication and mention of the grant of the patent: **04.03.2026**
(21) Application number: 20833498.7
(22) Date of filing: 29.05.2020
(51) Int. Cl.: A61K 9/107, A61K 47/24, A61K 38/17, A61K 38/18, A61P 43/00, A61K 9/00

(54) **PHARMACEUTICAL FORMULATION OF NON-ACTIVATED POLYPEPTIDE TRP**
PHARMAZEUTISCHE FORMULIERUNG VON NICHT-AKTIVIERTEM POLYPEPTID-TRP
FORMULATION PHARMACEUTIQUE DE POLYPEPTIDE TRP NON ACTIVÉ

(30) Priority: 26.06.2019 KR 20190076443
(43) Date of publication of application: 04.05.2022
(73) Proprietor: Met Life Sciences Co., Ltd., Seoul 03722 (KR)
(72) Inventor: YOOK, Jong In, Seoul 06004 (KR)
(74) Representative: Mewburn Ellis LLP
(86) International application number: PCT/KR2020/007011
(87) International publication number: WO 2020/262827

(56) References cited:
- WO-A1-94/08623
- KR-A- 20060 106 606
- US-A1- 2008 064 065
- XING, Y. ET AL.: "Lysophosphatidylcholine modulates the aggregation of human islet amyloid polypeptide", PHYSICAL CHEMISTRY CHEMICAL PHYSICS, vol. 19, 2017, pages 30627 - 30635, XP055778353
- GUO, J. ET AL.: "Pharmacokinetic behavior of cyclosporin A in rabbits by oral administration of lecithin vesicle and sandimmun neoral.", INTERNATIONAL JOURNAL OF PHARMACEUTICS., vol. 216, 2001, pages 17 - 21, XP027380042
- KIM, N. H. ET AL.: "A platform technique for growth factor delivery with novel mode of action.", BIOMATERIALS, vol. 35, 2014, pages 9888 - 9896, XP029070184, DOI: 10.1016/j.biomaterials.2014.08.005

## Description

### [Technical Field]

The present invention relates to a pharmaceutical formulation including a non-activated polypeptide TRP, and more particularly to a pharmaceutical formulation including a non-activated polypeptide TRP and a phospholipid dispersant.

### [Background Art]

A non-activated polypeptide (TRP: tissue-regenerative polypeptide) is a material having a new mechanism capable of promoting formation or regeneration of tissues such as bone, cartilage, and the like, or preventing fibrosis and hardening of organs such as the kidneys, liver, lungs, heart, and the like, and has a structure containing a PTD (protein transduction domain), which enables cell membrane permeation without the aid of a cell membrane receptor, an FAD (furin activation domain), having at least one proprotein convertase cleavage site and cleaved with the proprotein convertase to activate a non-activated TRD (tissue regeneration domain) in a cell, and a TRD, which is activated through cleavage with the proprotein convertase of FAD to promote tissue growth or formation in a cell or induce tissue regeneration (Korean Patent No. 10-00775958 and U.S. Patent No. 8,268,590: the patent publication cited as D1 in the European search report).

Non-activated TRP has a structure and properties completely different from those of conventionally known active proteins such as rhBMP or TGF-β. Specifically, all conventionally known rhBMPs and TGF-βs are biochemically activated before being introduced into human or mammalian cells, and have a three-dimensional structure (Eur. J. Biochem., 237:295, 1996; J. Mol. Biol., 287:103, 1999).

Therefore, in order to produce rhBMPs and TGF-βs, which are conventional activators, there is a problem in that it is necessary to rely on recombinant culture of mammalian animal cells such as CHO and the like, which have remarkably low productivity. Some rhBMP, such as rhBMP14 (MP-52) from BioPharm, is produced by culturing inexpensive recombinant *E. coli* instead of animal cells, but even in this case, there are many restrictions on the biochemical structure of the BMP to be produced. Specifically, active MP-52 may be produced using recombinant *E. coli,* but other active BMPs or active TGF-βs that may be administered into the human body for promoting bone or cartilage formation and regenerating various tissues, such as active rhBMP2, rhBMP4, rhBMP7, etc., are not easy to produce using recombinant *E. coli,* and a tertiary or quaternary structure thereof is required for pharmacological effects. Moreover, these conventional recombinant growth factors are sensitive to temperature changes, and have disadvantages of low stability at room temperature (Table 1 (comparison of differences between recombinant growth factor and TRP)).

In contrast, non-activated TRP exists in the form of a weakly soluble inclusion body, and thus has very high stability even at room temperature and is very robust even with changes in acidity (Table 1).

**[Table 1]**

| **Classification** | **Conventional recombinant growth factor** | **TRP** |
|---|---|---|
| Production | CHO cell or *E. coli* | *E. coli* |
| | Refolding from *E. coli* protein | Denaturation of inclusion body |
| Production cost | Expensive, difficulty in mass production | Low production cost, easy mass production |
| Apparatus | Requiring large-scale facility and apparatus | Very simple facility and apparatus |
| Product features | Soluble growth factor having biological activity | Denatured polypeptide having no biological activity |
| Tertiary and quaternary structure of protein | Tertiary and quaternary structures essential for pharmacological effects | Peptide having primary structure alone |
| | Existing in nature | Biological metamaterial |
| Solubility | Soluble | **Weakly soluble** |
| Stability and storage | Low room-temperature stability, sensitive to temperature changes | Very stable to changes in temperature and acidity |
| Biological mechanism of action (MoA) | Direct binding to growth factor receptor | Transformation into active growth factor after intracellular permeation regardless of receptor (prodrug) |
| Potency | mg scale | µg scale, at least 100 times as effective in humans and animals |
| Administration method | Use after hydration to carrier | Possible to develop prefilled syringe or various formulations |

However, in order to use non-activated TRP as a therapeutic agent, there has conventionally been a problem in that it is necessary to improve dispersibility through inhibition of aggregation and precipitation due to poor solubility of TRP, and to overcome low cell permeability and cytotoxicity when administered at a high dose. Moreover, when delivering a protein into a cell using PTD, the size of the protein delivery system (cargo) is very limited, and it is difficult to deliver a protein of 10 kd or more (J. Cell Sci., 129:893-897, 2016). For example, TRD and FAD in the TRP correspond to protein cargos, and BMP-2 is 44.7 kd, BMP-7 is 49.3 kd, and HGF is 83.1 kd, so intracellular permeation of the therapeutic protein using PTD is very limited. Therefore, when methods for preventing the aggregation of hydrophobic proteins and increasing the intracellular permeability of large-sized cargo are developed, it will be possible to increase the effect of TRP on treating diseases.

Accordingly, the present inventors have made great efforts to develop a new formulation for using non-activated TRP for therapeutic purposes, and thus ascertained that, when non-activated TRP is used along with a dispersant as defined in claim 1, (1) TRP aggregation is inhibited, (2) the cell permeability of TRP, having a large protein cargo size, is increased, (3) cytotoxicity is reduced, (4) the stability of the protein is increased, and (5) the therapeutic effect is also improved, thus culminating in the present invention.

### [Disclosure]

It is an object of the present invention to provide a pharmaceutical formulation capable of ensuring dispersibility through inhibition of aggregation of non-activated TRP, increasing the stability of protein medicines, and improving cell permeability, thus increasing therapeutic efficiency.

It is another object of the present invention to provide the pharmaceutical formulation for use in the treatment of fibrotic disease.

Disclosed herein, but not as a claimed invention, is a method of treating or preventing fibrotic disease including administering a composition including TRP2 and a phospholipid dispersant.

Disclosed herein, but not as a claimed invention, is the use of a composition including TRP2 and a phospholipid dispersant for the treatment or prevention of fibrotic disease.

Disclosed herein, but not as a claimed invention, is the use of a composition including TRP2 and a phospholipid dispersant for the manufacture of a medicament for the treatment or prevention of fibrotic disease.
In order to accomplish the above objects, the present invention provides a pharmaceutical formulation, comprising;
(a) a non-activated polypeptide TRP(tissue regenerative polypeptide) containing a PTD (protein transduction domain), which enables cell membrane permeation without aid of a cell membrane receptor, an FAD (furin activation domain), having at least one proprotein convertase cleavage site that can be cleaved with a proprotein convertase to activate a non-activated TRD (tissue regeneration domain) in a cell, and a TRD, which is activated through cleavage with the proprotein convertase of FAD to promote tissue growth or formation in a cell or induce tissue regeneration; and
(b) a dispersant selected from the group consisting of lecithin, Cremophor(CAS number 61791-12-6) and glyceryl trioleate,
wherein in the non-activated polypeptide TRP(tissue regenerative polypeptide) is TRP2(tissue regenerative polypeptide 2) represented by the amino acid sequence of SEQ ID NO:28.

In addition, the present invention provides the pharmaceutical formulation for use in the treatment of fibrotic disease including 100 parts by weight of the non-activated polypeptide TRP and 100 to 50,000 parts by weight of the phospholipid dispersant.

Disclosed herein, but not as a claimed invention, is method of treating or preventing fibrotic disease including administering a composition including 100 parts by weight of TRP2 and 100 to 50,000 parts by weight of a phospholipid dispersant.

Disclosed herein, but not as a claimed invention, is the use of a composition including 100 parts by weight of TRP2 and 100 to 50,000 parts by weight of a phospholipid dispersant for the treatment or prevention of fibrotic disease.

Disclosed herein, but not as a claimed invention, is the use of a composition including 100 parts by weight of TRP2 and 100 to 50,000 parts by weight of a phospholipid dispersant for the manufacture of a medicament for the treatment or prevention of fibrotic disease.

### [Description of Drawings]

FIG. 1 shows the results of DLS analysis of TRP (A) and of confirmation of intracellular permeability thereof using a confocal fluorescence microscope (B and C);
FIG. 2 shows results confirming, through DLS, the aggregation of TRP when a dispersant is not used (B) and the effect of dispersion of TRP2 using a dispersant;
FIG. 3 shows scanning electron microscope images of the particles of TRP2 when a dispersant is not used (TRP2) and the particles of TRP2 dispersed using egg lecithin (TRP2-EL), soybean lecithin (TRP2-SL), LysoPC (TRP-LysoPC), triglycerin (TRP2-Glyceryl Trioleate), and DSPE-PEG-amine (TRP2-DSPE-PEG-amine);
FIG. 4 shows images of TRP used in Example 3 and TRP2 in the dispersion formulation observed in three dimensions using a confocal microscope after treating 293 cells therewith for 4 hours (upper images), and images confirming the distribution of TRP2 through Z-stacks (lower images);
FIG. 5 shows images confirming intracellular permeability of TRP used in Example 3 and TRP2 in the dispersion formulation, taken using a transmission electron microscope after treating 293 cells therewith for 4 hours;
FIG. 6 shows images of TRP used in Example 3 and TRP2 in the dispersion formulation observed for the endosome marker Rab7-mCherry fluorescence using a confocal microscope after treating 293 cells therewith for 4 hours;
FIG. 7 shows results confirming the cytotoxicity of the TRP formulation including a phospholipid emulsifier as a dispersant;
FIG. 8 shows results confirming the rapid aggregation of TRP when not using a dispersant (A) and the results of DLS measurement of the aggregation inhibitory effect when using various dispersants depending on the time and storage temperature (B to D);
FIG. 9 shows the results of DLS measurement of the stability of TRP2 depending on changes in the concentration of egg lecithin and depending on the storage time and temperature;
FIG. 10 shows the results of DLS measurement of the stability of TRP2 depending on the storage time and temperature after dispersing various amounts of TRP2 using egg lecithin;
FIG. 11 shows confocal microscope images (A) and results of western blotting (B) on the intracellular permeability of TRP2 stored in a refrigerated state for 108 days after being dispersed in various methods using egg lecithin;
FIG. 12 shows the results of observation of the efficacy of inhibition of peritoneal fibrosis depending on the dose of TRP2 not including a dispersant in *in-vivo* animal models;
FIG. 13 shows the results of observation of the efficacy of inhibition of peritoneal fibrosis depending on the dose of the TRP2 formulation including Cremophor in *in-vivo* animal models;
FIG. 14 shows the results of observation of the efficacy of inhibition of peritoneal fibrosis depending on the dose of the TRP2 formulation including egg lecithin in *in-vivo* animal models;
FIG. 15 shows the results of observation of the efficacy of inhibition of peritoneal fibrosis depending on the dose of the TRP2 formulation including soybean lecithin in *in-vivo* animal models; and
FIG. 16 shows 50% effective dose (ED50) results based on the results of experiments on the use of TRP2 not including a dispersant and TRP2 including a dispersant on animals.

### [Mode for Invention]

Unless otherwise defined, all technical and scientific terms used herein have the same meanings as those typically understood by those skilled in the art to which the present invention belongs. Generally, the nomenclature used herein is well known in the art and is typical.

The non-activated polypeptide (TRP: tissue-regenerative polypeptide) of the present invention has a structure containing a PTD (protein transduction domain), which enables cell membrane permeation without the aid of a cell membrane receptor, an FAD (furin activation domain), having at least one proprotein convertase cleavage site and cleaved with the proprotein convertase to activate a non-activated TRD (tissue regeneration domain) in a cell, and a TRD, which is activated through cleavage with the proprotein convertase of FAD to promote tissue growth or formation in a cell or induce tissue regeneration, and is a material having a new mechanism capable of promoting the formation or regeneration of tissues such as bone, cartilage, and the like, or preventing fibrosis and hardening of organs such as the kidneys, liver, lungs, heart, and the like (Korean Patent No. 10-00775958 and U.S. Patent No. 8,268,590).

Non-activated TRP exists in the form of a weakly soluble inclusion body, so it has very high stability even at room temperature and is very robust even with changes in acidity. In order to use non-activated TRP as a therapeutic agent, aggregation and precipitation due to the poor solubility of TRP should be suppressed, and low cell permeability and cytotoxicity when administered at a high dose should be overcome.

TRP is a material having a mechanism that permeates cells and is activated through refolding and processing in the cytoplasm. In order to increase the pharmacological effect thereof, it is absolutely necessary to ensure physicochemical uniformity by inhibiting aggregation of a peptide preparation and to increase the intracellular permeability of a protein having a very large molecular weight (~50 kd) such as TRP2.

In the present invention, with the goal of developing a formulation that inhibits the aggregation of non-activated TRP, the TRP aggregation inhibitory effect of various aggregation inhibitors and dispersants was confirmed. As a result, a TRP formulation containing a phospholipid dispersant such as egg lecithin, soybean lecithin, triglyceride (glyceryl trioleate), LysoPC (L-α-lysophosphatidylcholine), Cremophor, etc. is confirmed to impart a high aggregation inhibitory effect, improve cell permeability of TRP, reduce cytotoxicity, and increase *in-vivo* efficacy.

Accordingly, an aspect of the present invention pertains to a pharmaceutical formulation including (a) a non-activated polypeptide TRP (tissue regenerative polypeptide) containing a PTD (protein transduction domain), which enables cell membrane permeation without the aid of a cell membrane receptor, an FAD (furin activation domain), having at least one proprotein convertase cleavage site that can be cleaved with the proprotein convertase to activate a non-activated TRD (tissue regeneration domain) in a cell, and a TRD, which is activated through cleavage with the proprotein convertase of FAD to promote tissue growth or formation in a cell or induce tissue regeneration, and (b) a dispersant selected from the group consisting of lecithin, Cremophor(CAS number 61791-12-6) and glyceryl trioleate,
wherein in the non-activated polypeptide TRP(tissue regenerative polypeptide) is TRP2(tissue regenerative polypeptide 2) represented by the amino acid sequence of SEQ ID NO:28.

In the present invention, the lecithin may be egg lecithin, soybean lecithin, sunflower oil lecithin, canola lecithin, cottonseed lecithin, or lecithin extracted from animal fat, but is not limited thereto. Preferably, egg lecithin or soybean lecithin is used, and egg lecithin is more preferably used.

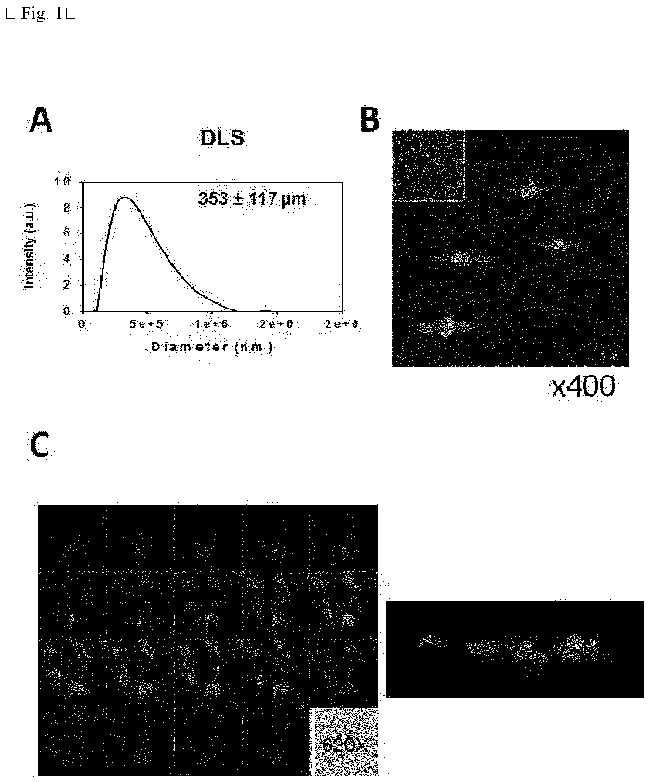

In an embodiment of the present invention, the aggregation of TRP was confirmed through DLS (dynamic light scattering, Otsuka, ELSZ-2000Zs) and confocal fluorescence microscopy. Most of the TRP particles existed at a size of 300 µm or more due to aggregation, and were observed in very large aggregates, even when using a confocal microscope. Therefore, these particles exhibited low intracellular permeation efficiency and mostly tended to exist outside the cell membrane (FIG. 1).

TRP is a material having a mechanism that permeates cells and is activated through refolding and processing in the cytoplasm. In order to increase the pharmacological effect thereof, it was confirmed that it was necessary to ensure physicochemical uniformity by inhibiting the aggregation of a peptide preparation and to increase the intracellular permeability of a protein having a very large molecular weight (~50 kd) such as TRP2.

Since normal cells have a size of 10-20 µm, it is essential to make a formulation having a uniform size of 5 µm or less in order to use TRP for therapeutic purposes. From a pharmacological point of view for patient treatment, a formulation having a uniform size of 0.5 µm or less is preferred.

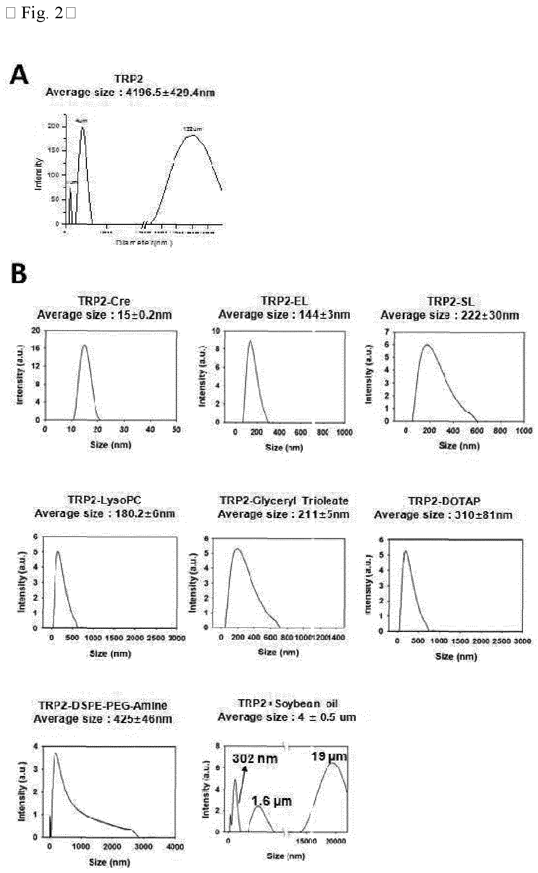

In another embodiment of the present invention, the TRP2 aggregation inhibitory effect was confirmed using sugars, amino acids, ammonium sulfate, and emulsifiers, which have conventionally been used to formulate peptides or antibodies. It was confirmed for the conventional aggregation inhibitor that the TRP aggregation inhibitory effect was insignificant (Table 2), and when 10% glycerol was used, an effect of slightly reducing the size of aggregates of TRP was confirmed. Even in this case, however, TRP was mostly present in a very large size, and TRP existed outside the cell membrane rather than permeating cells (FIG. 2).

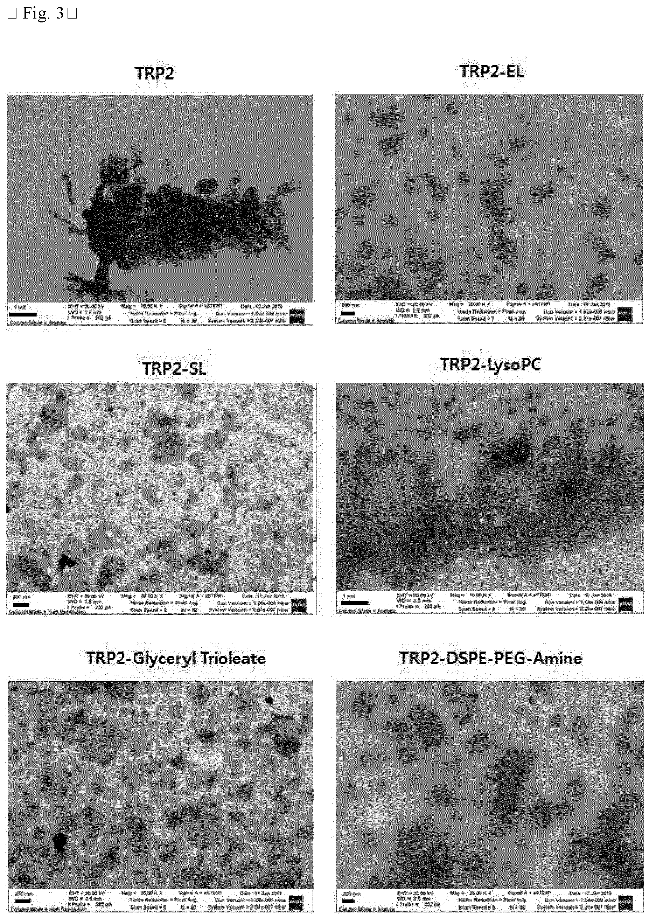

In another embodiment of the present invention, the TRP aggregation inhibitory effect of a TRP formulation including a dispersant such as Cremophor, egg lecithin, soybean lecithin LysoPC (lysophosphatidylcholines), glyceryl trioleate, DOTAP, DSPE-PEG-amine, and soybean oil was confirmed, and a phospholipid emulsifier such as Cremophor, egg lecithin, soybean lecithin, LysoPC, and glyceryl trioleate effectively inhibited the aggregation of TRP2 and maintained TRP2 in the form of micelles. However, it was confirmed that soybean oil, which is mainly used for weakly soluble low-molecular-weight compounds, had no aggregation inhibitory effect (Table 3 and FIGS. 2 and 3).

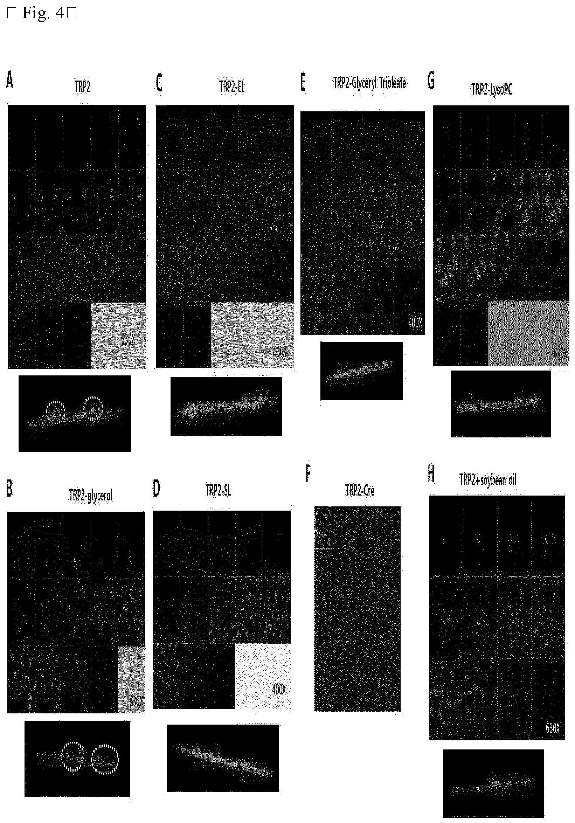

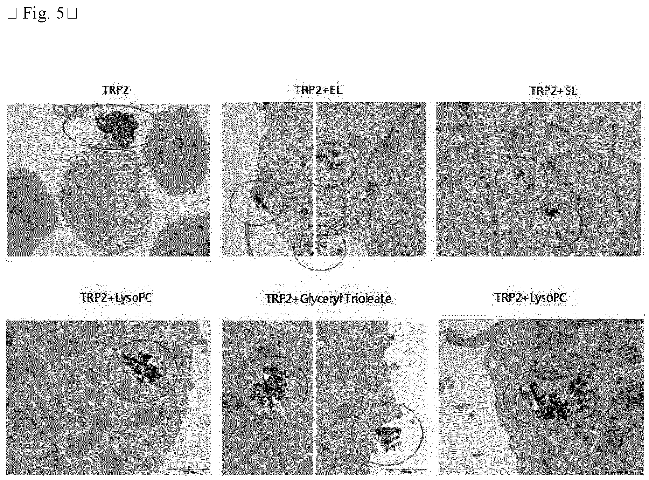

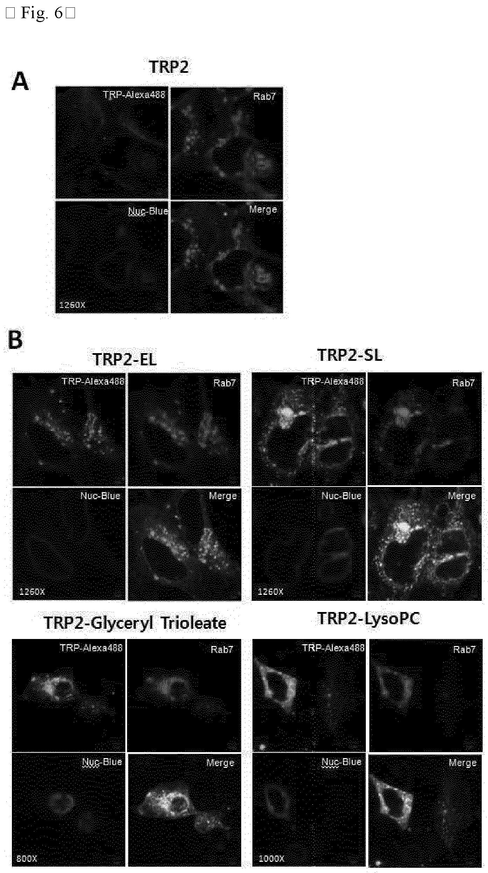

In another embodiment of the present invention, with regard to Cremophor, egg lecithin, soybean lecithin, LysoPC, and glyceryl trioleate, which were confirmed to exhibit an aggregation inhibitory effect, the effect of increasing cell membrane permeability of TRP was confirmed through confocal fluorescence microscopy and transmission electron microscopy. For cells administered with only TRP, TRP was mostly present on the cell membrane, but a TRP2 formulation added with an emulsifier (Cremophor, egg lecithin (EL), soybean lecithin (SL), LysoPC, and glyceryl trioleate) took a generally homogeneous form due to the reduced particle size, so it passed through the cell membrane and was thus observed in the cytoplasm (FIGS. 4 and 5), indicating that the cell membrane permeability of TRP was greatly increased by reducing the particle size of the TRP2 formulation due to the aggregation inhibitory effect. It was confirmed that the TRP2 formulation containing such a dispersant was effectively delivered into the cytoplasm through endosomes (FIG. 6).

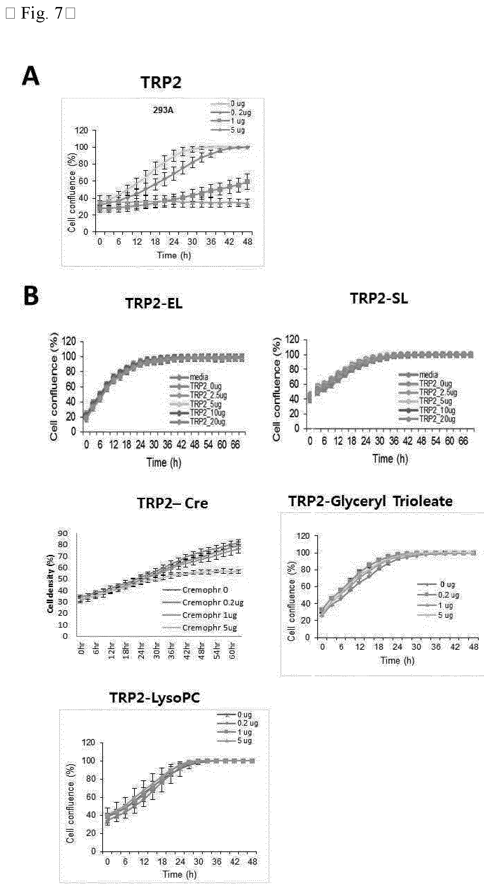

In another embodiment of the present invention, cytotoxicity of the TRP formulation including Cremophor or egg lecithin in 293A cells was analyzed using a JuLI Stage automated cell imaging system, and when the dispersant was not used, cytotoxicity appeared with an increase in the amount of the formulation that was used (1 µg and 5 µg), and egg lecithin (EL), soybean lecithin (SL), triglyceride (glyceryl trioleate), and LysoPC did not exhibit cytotoxicity even when the amount of the formulation that was used was increased (FIG. 7). Cremophor exhibited low cytotoxicity compared to when the dispersant was not used, but cytotoxicity appeared at a high dose (5 µg). Therefore, it was confirmed that egg lecithin, soybean lecithin (SL), triglyceride (glyceryl trioleate), and LysoPC were suitable for use in TRP formulations.

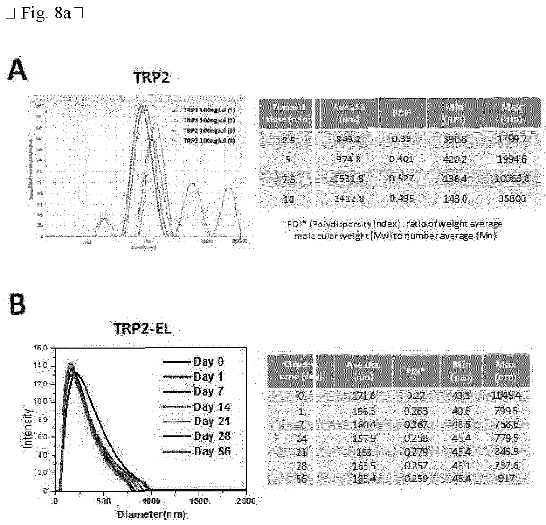

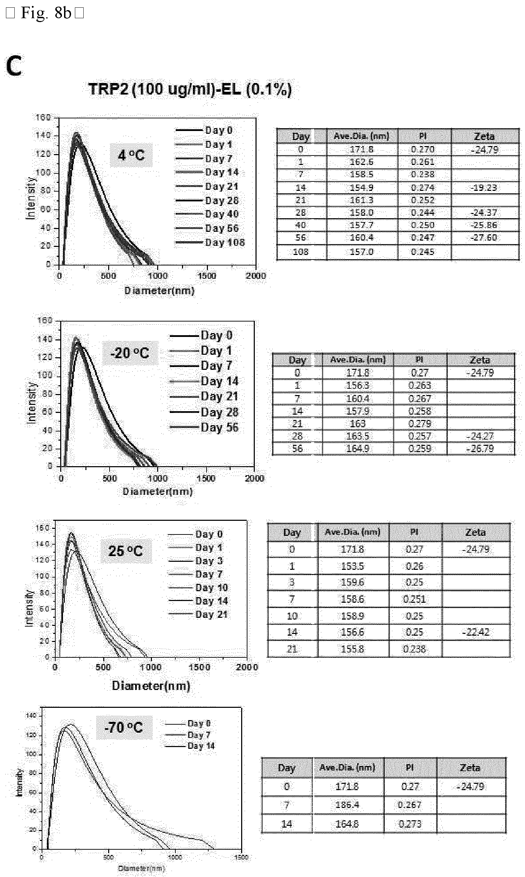

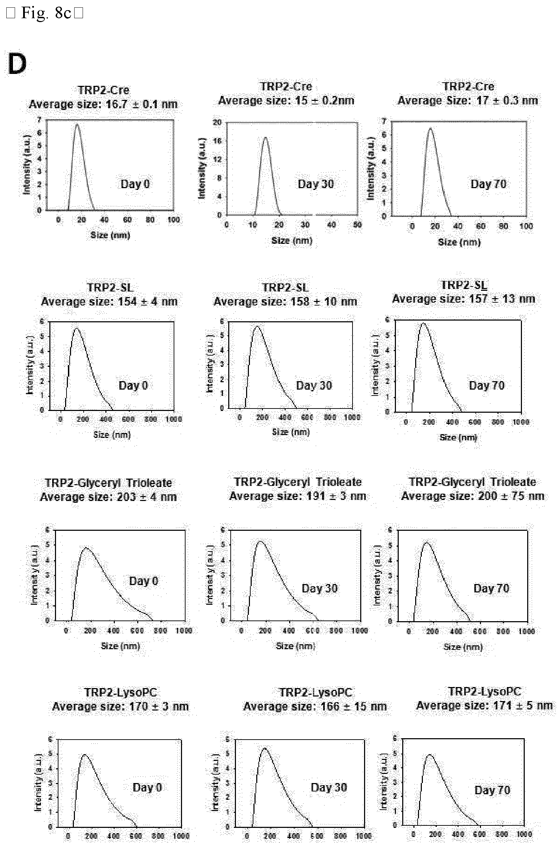

In another embodiment of the present invention, in order to confirm the stability of a formulation including TRP2 and egg lecithin, the formulation was frozen at -20°C and then allowed to stand at room temperature to evaluate whether the aggregation inhibitory ability is maintained over time. TRP2 not including a dispersant rapidly aggregated within a few minutes, and the size of the aggregate increased over time. The average size of the TRP2 formulation containing egg lecithin was 150 nm or less even after 56 days, indicating that the aggregation inhibitory ability was maintained constant (FIG. 8). In addition, it was confirmed that the formulation including a dispersant was stable for a very long time at various storage temperatures of TRP.

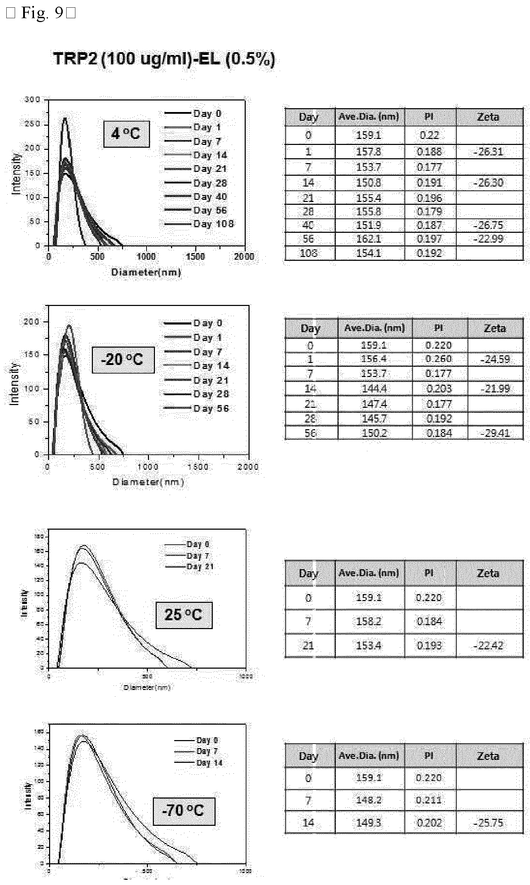

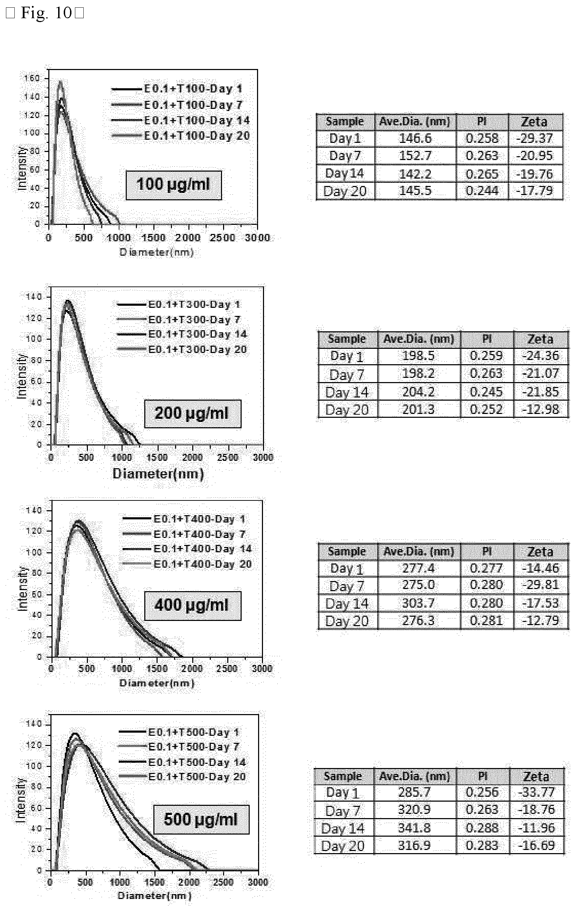

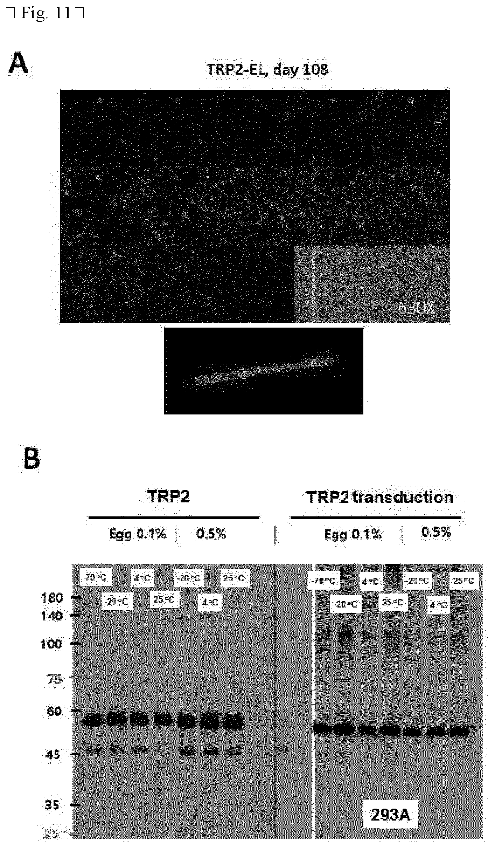

In another embodiment of the present invention, when egg lecithin was used at various concentrations and when TRP2 was used at various concentrations, it was confirmed that dispersibility was maintained depending on storage conditions and long-term storage (FIGS. 9 and 10). Moreover, it was confirmed that the increased intracellular permeability of TRP2 including the dispersant was maintained under various storage conditions (FIG. 11).

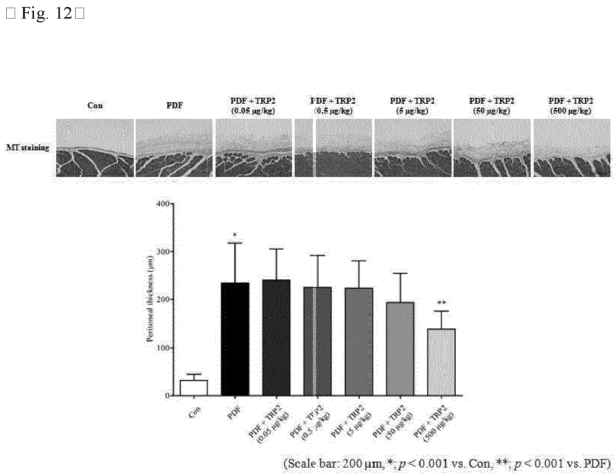

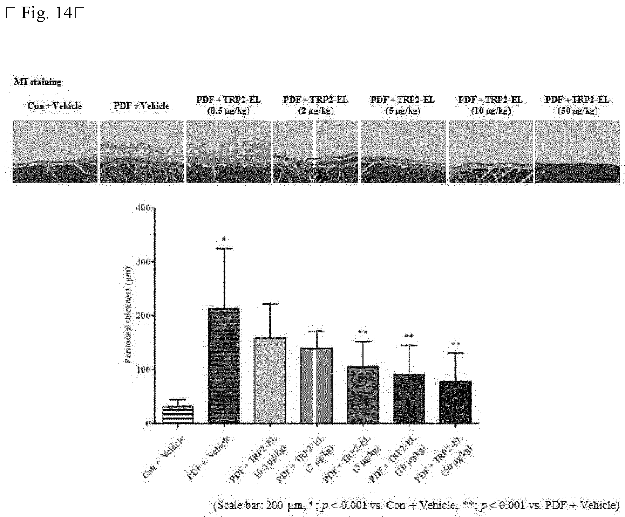

In another embodiment of the present invention, when the TRP formulation including the dispersant (Cremophor, egg lecithin or soybean lecithin) was administered to an animal model, the *in-vivo* effect of the TRP2 formulation including the dispersant in a peritoneal dialysis (PD) animal model was confirmed. With regard to the inhibition of peritoneal fibrosis in animal models, the formulation including the dispersant showed very low ED50 compared to TRP2 not including a dispersant (Tables 5 to 9 and FIGS. 12 to 16).

The formulation of the present invention may be suitable for use in injection, and the dispersant, such as egg lecithin, soybean lecithin, triglyceride, or Cremophor, is included in an amount of 100 to 50,000 parts by weight based on 100 parts by weight of the non-activated polypeptide TRP, preferably 200 to 10,000 parts by weight based on 100 parts by weight of TRP, and more preferably 500 to 5,000 parts by weight based on 100 parts by weight of TRP.

The non-activated TRP of the present invention may be prepared in a manner in which a recombinant vector in which a nucleotide sequence encoding an FAD, a nucleotide sequence for a PTD, a nucleotide sequence for tagging, and a nucleotide sequence encoding four or more histidines for separation and purification are inserted upstream of 5' of DNA encoding a TRD is constructed, and bacteria transformed with the recombinant vector are cultured and thus a [PTD-FAD-TRD] polypeptide is expressed, after which the culture solution is added with a urea solution, thus removing two-dimensional and three-dimensional structures of the polypeptide or transforming the polypeptide into a one-dimensional linear structure, followed by purification of the [PTD-FAD-TRD] polypeptide. For example, it may be prepared through the method disclosed in Korean Patent Application Publication No. 2006-0106606.

In the present invention, the TRD is human BMP-7.

In the present invention, the FAD has a proprotein convertase cleavage site and is cleaved with the proprotein convertase in a cell to thus activate the TRD.

The PTD that is used in the present invention is TAT (YGRKKRRQRRR: SEQ ID NO: 27).

In the method of producing the non-activated TRP according to the present invention, the purification step includes binding the polypeptide to nickeltitanium beads, followed by washing with the same solution and then elution with imidazole and a high-salt buffer solution, but the present invention is not limited thereto.

In the present invention, the proprotein convertase may be furin, but is not limited thereto. Examples thereof may include PC7, PC5/6A, PC5/6B, PACE4, PC1/3, PC2, PC4, and the like.

The non-activated TRP according to the present invention does not have three-dimensional stereoregularity common to conventional active BMPs, and does not have biochemical activity by itself, but when administered in a human or mammalian body, the proprotein convertase cleavage site of FAD is cleaved with the proprotein convertase present in cells *in vivo* to thus activate the TRD, and the activated TRD is secreted out of the cells, thereby exhibiting the expected potency. The TRP according to the present invention preferably takes the form of a polypeptide in which PTD, FAD and TRD are fused. The TRP according to the present invention has the function of promoting the formation or regeneration of tissues such as bones, cartilage and the like in the human body, or preventing fibrosis and hardening of organs such as the kidneys, liver, lungs, heart and the like, and further induces regeneration of the original tissue.

The TRP according to the present invention may be mass-produced for practical use without being limited by the biochemical structure of proteins by culturing bacteria such as recombinant *E. coli,* and since the biochemically inactive state thereof is maintained before administration *in vivo,* the production cost thereof is only a few tenths of that of conventionally known active proteins for similar uses (rhBMPs, TGF-βs, etc.), and separation, purification, handling, storage, and administration thereof are extremely simple and convenient.

In addition, the non-activated TRP according to the present invention is a polypeptide containing a PTD, which enables cell membrane permeation without the aid of a cell membrane receptor, an FAD, having at least one proprotein convertase cleavage site that can be cleaved with the proprotein convertase to activate a non-activated TRD in the cell, and a TRD, which is activated in the cell through cleavage with the proprotein convertase of FAD to promote tissue growth or formation or tissue regeneration. Although the TRP has no activity by itself, it permeates organisms or cells, after which FAD is cleaved with the proprotein convertase present in a large amount in almost all cells, so the TRD is activated, and the activated protein is secreted out of the cells to thus exert the potency thereof. The TRP according to the present invention is capable of solving all problems with conventional active BMP-like proteins, which are expensive to prepare, difficult to store and handle, and essentially require a receptor.

The composition containing the non-activated TRP according to the present invention as an active ingredient is preferably prepared by mixing and diluting the active ingredient with a pharmaceutically acceptable excipient or matrix as a carrier or by enclosing the active ingredient in a carrier in a container form, depending on the administration method, dosage form, and therapeutic purpose. Moreover, the composition may be used in combination with other drugs that are beneficial for the treatment of other bone defects. Here, the preparation of a physiologically acceptable protein composition having a desired pH, isotonicity, stability, etc. may be performed using any typical method known in the art to which the present invention belongs.

In the present invention, preferable examples of the matrix include, depending on biocompatibility, biodegradability, mechanical properties, cosmetic appearance, and interface properties, biodegradable and chemical materials such as calcium sulfate, tricalcium phosphate, hydroxyapatite, polylactic acid or polyanhydride, biodegradable and biological materials such as bone or skin collagens, other pure proteins or cellular matrix components, non-biodegradable and chemical materials such as sintered hydroxyapatite, bioglass, aluminate or other ceramics, and combinations of the aforementioned materials, such as polylactic acid, hydroxyapatite, collagen and tricalcium phosphate. However, the present invention is not limited to the carriers described above.

In the present invention, examples of the excipient may include lactose, dextrose, sucrose, sorbitol, mannitol, calcium silicate, cellulose, methyl cellulose, microcrystalline cellulose, polyvinylpyrrolidone, magnesium stearate, water, methylhydroxybenzoate, propylhydroxybenzoate, talc, mineral oil, and the like.

Meanwhile, the composition containing the non-activated TRP according to the present invention is preferably used through encapsulation or injection in a viscous form for administration to a bone injury site. The dose of the composition according to the present invention is not limited because it may be adjusted in consideration of the type of excipient or carrier that is used, such as a matrix or the like, the weight of the patient's bone, the bone injury site, the condition of the injured bone, the patient's age, gender and diet, the extent of suspicion of infection, the administration time, and other clinical factors. However, for the typically known effective amount of the composition, an appropriate amount may be administered continuously or dividedly in consideration of bone weight, and additional administration may be determined while observing bone growth.

In another embodiment of the present invention, when the TRP formulation including a dispersant (Cremophor, egg lecithin, or soybean lecithin) was administered to animal models, the *in-vivo* effect of the TRP2 formulation including a dispersant in a peritoneal dialysis (PD) animal model was confirmed. With regard to the inhibition of peritoneal fibrosis in animal models, the formulation including the dispersant showed very low ED50 compared to TRP2 not including a dispersant (Tables 5 to 9 and FIGS. 12 to 16).

Accordingly, another aspect of the present invention pertains to the pharmaceutical formulation for use in the treatment of fibrotic disease including 100 parts by weight of TRP2 and 100 to 50,000 parts by weight of the dispersant.

Disclosed herein, but not as a claimed invention, is a method of treating or preventing fibrotic disease including administering a composition including 100 parts by weight of TRP2 and 100 to 50,000 parts by weight of a phospholipid dispersant.

Yet another aspect of the present invention pertains to the use of a composition including 100 parts by weight of TRP2 and 100 to 50,000 parts by weight of a phospholipid dispersant for the treatment or prevention of fibrotic disease.

Disclosed herein, but not as a claimed invention, is the use of a composition including 100 parts by weight of TRP2 and 100 to 50,000 parts by weight of a phospholipid dispersant for the manufacture of a medicament for the treatment or prevention of fibrotic disease.

In the present invention, the fibrotic disease may include liver fibrosis, muscle fibrosis, lung fibrosis, kidney fibrosis, cardiac fibrosis, peritoneal fibrosis, various eye diseases caused by fibrosis, and the like. This fibrosis inhibitory effect may be applied to various cancers such as liver cancer, as well as chronic diseases. Moreover, since BMP-7 inhibits TGF-beta signaling (Nature Med. 2003, 9, 964-968), it may be used in combination with an immune checkpoint anticancer drug such as a PD1/PDL-1 antibody (Nature 2018, 554, 538- 543). It may also be used for various metabolic diseases, inflammation, hyperphosphatemia, and Alport syndrome.

### Examples

A better understanding of the present invention may be obtained through the following examples. These examples are merely set forth to illustrate the present invention, and are not to be construed as limiting the scope of the present invention, as will be apparent to those skilled in the art.

Particularly, in the following examples, only hBMP2 and hBMP7 are exemplified as the TRD constituting the TRP, but it will be apparent to those skilled in the art that not only human-derived BMPs such as hBMP3, hBMP4, hBMP6, and hBMP14 (MP-52), but also BMPs derived from mammals such as rats, cattle, pigs and the like, and other higher animals may also be used. In addition to BMPs, it will be apparent to those skilled in the art that it is also possible to use polypeptides, such as TGF-β superfamily, β-NGF (β-nerve growth factor), β-amyloid, ADAMs (a disintegrin and metalloproteinase-like), TNF-α family including ectodysplasin-A (Eda-1), MMPs including MT1-MMP (membrane type-matrix metalloproteinase) and MMP-2, and insulin-like growth factor-1 (IGF-1).

Also, in the following examples, only the prodomain of BMP is exemplified as FAD constituting TRP, but it will be apparent to those skilled in the art that the FAD is not limited, so long as it has a proprotein convertase cleavage site and is cleaved with the proprotein convertase in the cell to activate the TRD.

Also, in the following examples, the TRP protein was dispersed using a phospholipid and ultrasound, but it will be apparent to those skilled in the art that high-pressure homogenizers or dispersers commonly used for drug manufacture may be used.

### Example 1: Confirmation of TRP aggregation and cell permeability

In order to confirm the aggregation of TRP, DLS (dynamic light scattering, Otsuka, ELSZ-2000Zs) and confocal fluorescence microscopy were performed.

The TRP that was used was the following TRP2 having a BMP7 sequence as TRD.
TRP2 amino acid sequence (SEQ ID NO: 28):

MRGSHHHHHHGMASMTGGQQMGRDLYDDDDKDRWGSKLGYG
RKKRRQRRRGGLFGAIAGFIENGWEGMIDGGSTMAGTMHVRSLRAAAPHS
FVALWAPLFLLRSALADFSLDNEVHSSFIHRRLRSQERREMQREILSILGLPHRPRP
HLQGKHNSAPMFMLDLYNAMAVEEGGGPGGQGFSYPYKAVFSTQGPPLASLQDS
HFLTDADMVMSFVNLVEHDKEFFHPRYHHREFRFDLSKIPEGEAVTAAEFRIYKD
YIRERFDNETFRISVYQVLQEHLGRESDLFLLDSRTLWASEEGWLVFDITATSNHWV
VNPRHNLGLQLSVETLDGQSINPKLAGLIGRHGPQNKQPFMVAFFKATEVHFRSIR
STGSKQRSQNRSKTPKNQEALRMANVAENSSSDQRQACKKHELYVSFRDLG
WQDWIIAPEGYAAYYCEGECAFPLNSYMNATNHAIVQTLVHFINPETVPKPCC

APTQLNAISVLYFDDSSNVILKKY RNMVVRACGCHHYPYDVPDYA

Bold: PTD, Italic underlined: human BMP-7, Underlined: FAD

The particle size of TRP2 separated and purified from the inclusion body of transformed *E. coli* was measured using DLS. As a result, as shown in FIG. 1A, most of the TRP particles exhibited very large and various particle sizes due to aggregation, and in some cases, TRP existed at a size of 300 µm or more.

293A cells were treated with 100 ng of this TRP2 for 4 hours, and the cells were fixed in 10% formalin, stained with an X-press monoclonal antibody (Thermo Fisher R910-25) and a secondary antibody labeled with Alexa-594 (Thermo Fisher A32744) in order to observe TRP2, and observed using a confocal microscope (Carl Zeiss, LSM700).

Based on the results thereof, very large aggregates were observed even on the low-magnification confocal microscope of FIG. 1B, and multi-layer imaging and stereoscopic analysis (Z-stacks) were performed to observe the three-dimensional distribution pattern. Based on the results thereof, as shown in FIG. 1C, most TRP2 was present in an aggregated form outside the cell membrane, rather than inside the cells. Therefore, TRP2 exhibited low intracellular permeation efficiency, and tended to exist outside the cell membrane.

TRP is a material having a mechanism that permeates cells and is activated through refolding and processing in the cytoplasm. In order to increase the pharmacological effect thereof, it is necessary to ensure physicochemical uniformity by inhibiting the aggregation of a peptide preparation and to increase the intracellular permeability of a protein having a very large molecular weight (~50 kd) such as TRP2.

### Example 2: Analysis of TRP aggregation inhibitory effect using conventional peptide and antibody medicine formulations

The TRP2 aggregation inhibitory effect was evaluated using sugars, amino acids, ammonium sulfate, and emulsifiers, which have conventionally been used to formulate peptides or antibodies.

**Table 2]**

| | | Manufacturer | Catalog Number |
|---|---|---|---|
| Sugar | Glycerol (25%) | Amresco (VWR) | 0854 |
| | 3lucose (200%) | Amresco (VWR) | 0188 |
| | Fructose | Sigma | F0127 |
| | Sucrose | Sigma | S0389 |
| | Glycine | VWR | 0167 |
| | Histidine | Daejung | 1085-4425 |
| | Lysine monohydrochloride | Daejung | 5093-4125 |
| | Proline | Daejung | 6626-4125 |
| | Tyrosine | Daejung | 8575-4425 |
| Amino acid | Arginine hydrochloride | Daejung | 1144-4425 |
| | Valine | Daejung | 8647-4125 |
| | Leucine | Daejung | 5082-4125 |
| | Tryptophan | Daejung | 8619-4425 |
| | Alanine | Daejung | 1111-4125 |
| | Methionine | Daejung | 5666-4125 |
| Ammonium sulfate | AS | Amresco (VWR) | 97061-180 |
| Emulsifier (surfactant) | Polysorbate 20 (Tween 20) | Amresco (VWR) | 0777 |
| | Polysorbate 40 (Tween 40) | Sigma | P1504 |
| | Polysorbate 80 (Tween 80) | Amresco (VWR) | M126 |

To this end, the recombinant protein TRP2 at a concentration of 1 mg/ml in PBS was diluted with various dispersants at the concentrations shown in Table 2 to make 100 µg/ml of TRP2, which was then allowed to stand at room temperature for 1 hour, followed by DLS measurement. Here, the effect of each dispersant was evaluated in a semi-quantitative manner based on the average size of stabilized particles in DLS (0: > 4 µm; 1+: < 4 µm; 2+: < 3 µm; 3+: < 2 µm; 4+: < 1 µm; and 5+: 250 µm). Moreover, when it was determined that there was a dispersion effect, the stability of the particles was evaluated by additionally measuring Zeta potential.

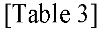

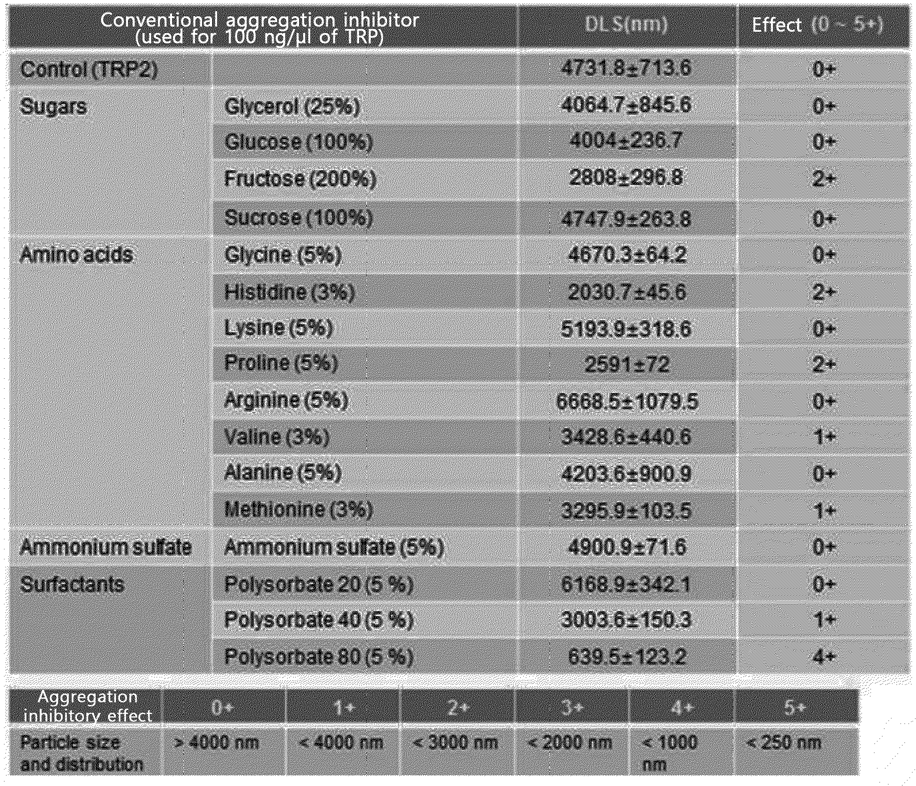

Based on the results thereof, as shown in Table 3, it was confirmed for the aggregation inhibitor conventionally used in recombinant proteins or antibodies that the TRP aggregation inhibitory effect was insignificant, and it was confirmed for Tween 80 that there was an aggregation effect, but that the Zeta potential was very low (1.63 mV), indicative of very poor particle stability.

Moreover, 10% glycerol, which is most commonly used to prevent aggregation of recombinant proteins or antibodies, slightly reduced the aggregation size of TRP, but even in this case, TRP2 was mostly present in a very large size, from which it was found that 10% glycerol was unsuitable for use as the main dispersant of TRP2.

### Example 3: Analysis of aggregation inhibitory effect of TRP formulation using phospholipid emulsifier as dispersant

The TRP aggregation inhibitory effect of TRP formulations including, as an emulsifier, Cremophor EL (Merck, 238470), egg lecithin (BOC sciences, BS18J04211), soybean lecithin (TCI, L0023), LysoPC (lysophosphatidylcholines) (Avanti, 830071P), glyceryl trioleate (Sigma, T7140), DOTAP (Avanti, 890890P), DSPE-PEG-Amine (Avanti, 880128P), and soybean oil (CJ Cheiljedang) was evaluated.

To this end, the recombinant protein TRP2 at a concentration of 1 mg/ml in PBS was sonicated in a 10% glycerol solution and dispersed using various dispersants shown in Table 3, and then the formation of micelles thereof was induced using weak ultrasound. Here, the appropriate concentration of the dispersion emulsifier was determined using an emulsifier having a concentration of 0.1%. The dispersed TRP2 was allowed to stand at room temperature for 1 hour to induce aggregation, and the average size and dispersion of the particles were measured through DLS. Moreover, the aggregation inhibitory effect of these dispersants was observed using a scanning electron microscope (Carl Zeiss, model name: Merlin).

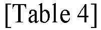

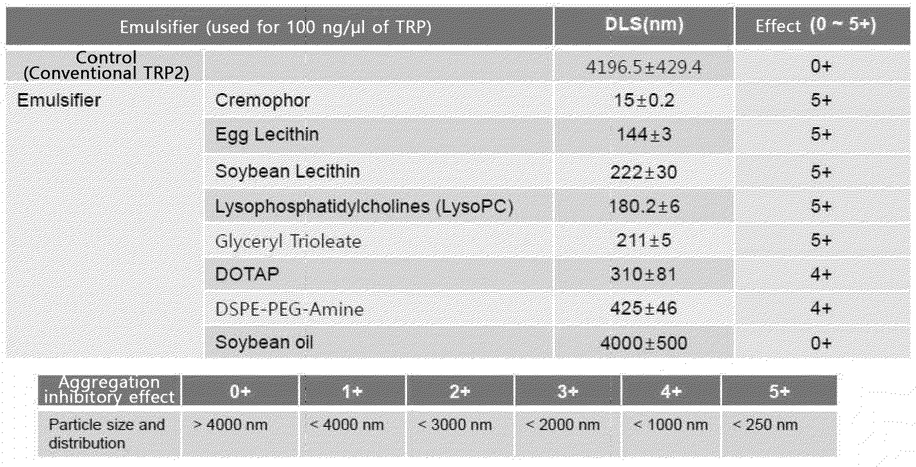

Based on the results thereof, as shown in Table 4 and in FIG. 2, upon dispersion using, as the phospholipid emulsifier, 10% Cremophor (TRP-Cre), 0.1% egg lecithin (TRP2-EL), soybean lecithin (TRP2-SL), LysoPC (TRP2-LysoPC), or glyceryl trioleate (TRP2-Glyceryl trioleate), the aggregation of TRP2 was effectively inhibited, and the form of micelles having an average size of 250 nm or less was maintained, thereby exhibiting an excellent dispersion effect, as measured through DLS. However, it was confirmed that soybean oil, which is mainly used for weakly soluble low-molecular-weight compounds, has no aggregation inhibitory effect.

In addition, as shown in FIG. 3, stable dispersion at a certain size using the phospholipid emulsifier was directly confirmed through scanning electron microscopy (TRP2/TRP2-LysoPC formulation x10,000, with a scale increment of 1 µm; other formulations x20,000, with a scale increment of 20 nm).

Therefore, it was confirmed that the phospholipid emulsifier can be used as an aggregation inhibitor and dispersant capable of effectively dispersing TRP, unlike the conventional recombinant protein or antibody.

### Example 4: Analysis of effect of increasing cell membrane permeability of TRP formulation including dispersant

TRP was developed as a precursor protein prodrug of BMP-7. Therefore, in order for TRP to exhibit effective pharmacological activity, it is very important that it be effectively delivered into the cytoplasm through endosomal transport. Thus, an attempt was made to confirm that the formulation showing the excellent dispersion effect of TRP2 enhances the intracytoplasmic permeability of TRP2. With regard to Cremophor, egg lecithin, soybean lecithin, LysoPC, and glyceryl trioleate, which were confirmed to exhibit the aggregation inhibitory effect in Example 3, the effect of enhancing cell membrane permeability of TRP was evaluated through confocal fluorescence microscopy. To this end, egg lecithin (EL), soybean lecithin (SL), LysoPC, and glyceryl trioleate dispersants were prepared to a concentration of 0.1%, and Cremophor (Cre) was used at 10%. TRP2 was dispersed using each dispersant and ultrasound such that the concentration of TRP2 was 100 µg/ml, and 293A cells were treated therewith for 6 hours, fixed, and stained using an X-press monoclonal antibody (Thermo Fisher R910-25) and a secondary antibody labeled with Alexa-594 (Thermo Fisher A32744) in the same manner as in Example 1, followed by multi-layer imaging and stereoscopic analysis using a confocal microscope (Carl Zeiss, LSM700).

Based on the results thereof, as shown in FIG. 4A, in the case of TRP2 not including a dispersant, the aggregated protein was mostly attached to the outside of the cell membrane, similar to Example 1. When 25% glycerol was used as a dispersant, intracytoplasmic permeation was slightly increased, but the protein was mostly present outside the cell membrane, so there was no significant effect (FIG. 4B). However, for egg lecithin (TRP2-EL), soybean lecithin (TRP2-SL), glyceryl trioleate (TRP2-Glyceryl trioleate), Cremophor (TRP-Cre), and LysoPC (TRP2-LysoPC), which are phospholipid emulsifiers showing an excellent protein dispersion effect in Example 3, it was confirmed that a very large amount of protein was delivered into the cytoplasm (FIGS. 4C-G). However, soybean oil, which is commonly used for dispersing water-insoluble drugs, did not increase intracytoplasmic permeability (FIG. 4H).

In addition, in order to directly confirm the cell permeability of the TRP protein, the protein was stained with 2.5% uranyl acetate (Electron microscopy sciences, #22400) as in Example 1, and the protein was dispersed using each dispersion formulation. 293A cells were treated with 50 ng of TRP2 contained in each dispersion formulation, embedded in a resin, and observed using an electron microscope (JEOL, JEM1011).

Based on the results thereof, as shown in FIG. 5, TRP not including the dispersant (TRP2) was present in an aggregated form on the outer surface of the cell membrane, and when the dispersion formulation was used, it was confirmed that the protein was well delivered into the cytoplasm through endosomes (represented as red circles).

These results indicate that the TRP2 formulation containing Cremophor, egg lecithin, soybean lecithin, LysoPC, or glyceryl trioleate significantly improved the cell membrane permeability of TRP, as well as the aggregation inhibitory effect.

### Example 5: Confirmation of effect of TRP formulation including dispersant on increasing intracellular protein delivery using endosome

It was confirmed in Examples 3 and 4 that the phospholipid dispersant significantly increased the delivery of TRP2 into cells, as well as the aggregation inhibitory effect thereof. Moreover, it could be observed from the scanning electron micrographs that the TRP delivered into the cytoplasm was surrounded by a twolayered membrane structure. This suggests the possibility that efficient intracytoplasmic delivery of TRP2 using the phospholipid dispersant will utilize the endosomal pathway. To this end, the TRP protein was fluorescently labeled using an Alexa Fluor 488 (excitation: 494 nm, emission: 519 nm) protein labeling kit (Invitrogen, MP10235) according to the manufacturer's instructions. The fluorescently labeled TRP was dispersed and formed into micelles as in Example 4 using egg lecithin (TRP2-EL), soybean lecithin (TRP2-SL), glyceryl trioleate (TRP2-Glyceryl trioleate), and LysoPC (TRP2-LysoPC). Moreover, in order to detect endosomes by fluorescence, a fusion expression vector of an mCherry (excitation: 587 nm, emission: 610 nm) fluorescence gene and an endosome marker Rab7a (Rasrelated protein Rab-7a) gene was inserted into pcDNA3.1 (Invitrogen) to construct an mCherry-Rab7a expression vector (SEQ ID NO: 29) containing mCherry fluorescence and human Rab7a genes.

The mCherry-Rab7a expression vector thus constructed was transfected into 293A cells to induce expression, and after 48 hours, the cells were treated for 4 hours with 100 ng of the fluorescently labeled TRP2 and observed using a confocal microscope. Here, the cell nucleus was stained with NucBlue (Invitrogen, NucBlue Live ReadyProbes, R37605).

Based on the results thereof, as shown in FIG. 6A, in the case of TRP2 not including the dispersant, a very small amount of TRP2 was delivered into the cytoplasm, which was found to have almost no association with the endosome marker Rab7 (a scale increment of 5 µm). However, it was found that TRP dispersed by the various phospholipid formulations was delivered in a large amount into the cytoplasm and was present at the same site as Rab7a (co-localization), similar to Example 4 (FIG. 6B). Therefore, it can be concluded that micellar dispersion using egg lecithin, soybean lecithin, glyceryl trioleate, and LysoPC enables TRP to be effectively delivered into the cytoplasm through the endosomes, unlike the conventional TRP2.

### Example 6: Analysis of cytotoxicity of TRP formulation including dispersant

It was found in Examples 1 and 4 that the water-insoluble TRP protein was present in the form of being attached to the cell membrane surface. Moreover, it was found in Example 5 that the micelles, formed using the dispersion formulation, were delivered into the cytoplasm through endosomes. Cytotoxicity occurs due to destruction of the cell membrane upon excessive administration of TRP not including the dispersant, whereas prevention of cell membrane damage through endosomal recycling becomes possible when using the dispersion formulation. Therefore, the cytotoxicity of each TRP formulation in 293A cells (Thermo Fisher, R70507) was analyzed using a JuLI Stage automated cell imaging system in order to confirm the cytotoxicity reduction effect due to the dispersant. Here, 5000 293A cells were spread in a 24-well plate, and the cells were allowed to adhere for 16 hours. Thereafter, the cells were treated with TRP2 dispersed in various forms at various concentrations, and cell proliferation was observed using a real-time microscope mounted on a JuLI Stage to analyze cytotoxicity.

Based on the results thereof, as shown in FIG. 7A, undispersed TRP2 caused cytotoxicity when used at a high dose of 1 µg or more in a 24-well cell culture plate having an area of about 2 cm². However, 0.25% egg lecithin (TRP2-EL), soybean lecithin (TRP2-SL), glyceryl trioleate (TRP2-Glyceryl trioleate), and LysoPC (TRP2-LysoPC) did not cause cytotoxicity even when used in an amount of 20 µg. However, TRP2 dispersed using 10% Cremophor exhibited slight toxicity when used in an amount of 5 µg or more. Therefore, it was confirmed that egg lecithin (TRP2-EL), soybean lecithin (TRP2-SL), glyceryl trioleate (TRP2-Glyceryl trioleate), and LysoPC (TRP2-LysoPC) reduced cytotoxicity.

### Example 7: Confirmation of stability of TRP formulation including egg lecithin

In protein medicines, protein stability is a very important issue affecting reproducibility, approval, safety, and drug efficacy. In order to confirm the stability of conventional TRP2, it was frozen at -20°C and allowed to stand at room temperature to evaluate whether the aggregation inhibitory ability was maintained over time. As a result, protein aggregation occurred very quickly (within 10 minutes) due to the aggregation effect depending on the hydrophobic physicochemical properties of TRP, described in Table 1 (FIG. 8A).

In order to evaluate the effect of the dispersion formulation on protein stability in addition to the dispersion formulation effect described in Examples 3 to 5, TRP2 (TRP2-EL) was dispersed using 0.1% egg lecithin, and the stability of the protein drug was measured through DLS over time.

Based on the results thereof, as shown in FIG. 8A, TRP2 dispersed using egg lecithin maintained a very stable state for 56 days or more.

Storage temperature has a great influence on the stability of protein medicines. In order to confirm this influence, TRP2 was dispersed at a concentration of 100 µg/ml using 0.1% egg lecithin, and the stability thereof depending on various storage temperatures and periods was measured.

Based on the results thereof, as shown in FIG. 8C, it was confirmed that a very stable formulation having an average particle size of 200 nm or less was maintained at various temperatures of -70°C to 25°C for various storage periods. In particular, it was confirmed that the dispersion effect of egg lecithin on inhibition of TRP2 protein aggregation was long-lasting and stable because a very stable form can be sustained for 108 days or more in a refrigerated state at 4°C.

In order to confirm the TRP2 stability of dispersants other than egg lecithin, TRP2 was dispersed using 10% Cremophor, 0.1% soybean lecithin (TRP2-SL), 0.5% glyceryl trioleate (TRP2-Glyceryl trioleate), and 0.5% LysoPC (TRP2-LysoPC), and the stability of the particles was measured through DLS immediately after dispersion (Day 0) and after storage in a refrigerated state at 4°C for 30 days and 70 days.

Based on the results thereof, as shown in FIG. 8D, it was found that each formulation maintained a very stable state for 70 days or more, similar to egg lecithin.

Egg lecithin is used at various concentrations in medicines. In order to confirm the stability of the formulation depending on the concentration of egg lecithin, TRP2 was dispersed at a concentration of 100 µg/ml using 0.5% egg lecithin, and the stability of the protein particles depending on various storage temperatures and periods was measured through DLS.

Based on the results thereof, as shown in FIG. 9, even when 0.5% egg lecithin was used, protein dispersibility was stable regardless of storage conditions, very similar to the results of FIG. 8C using 0.1% egg lecithin.

In addition, in order to confirm the aggregation inhibitory effect depending on the dose of protein medicine, TRP2 was dispersed at various concentrations of 100 to 500 µg/ml using 0.1% egg lecithin and stored in a refrigerated state at 4°C for various periods, and the egg lecithin dispersibility depending on the TRP concentration was measured.

Based on the results thereof, as shown in FIG. 10, when 0.1% egg lecithin was used, TRP was maintained in a very stable state for 20 days or more even at a concentration of 400 µg/ml. However, 500 µg/ml or more of TRP caused some aggregation after 7 days.

In order to evaluate whether the cell permeability of TRP is maintained even after long-term storage using the dispersant, 293A cells were treated for 4 hours with TRP2 dispersed using 0.1% egg lecithin and stored in a refrigerated state for 108 days, followed by observation using a confocal microscope.

Based on the results thereof, as shown in FIG. 11A, it was found that the intracellular permeability of TRP dispersed using egg lecithin and stored for 108 days was maintained without change. Moreover, in order to evaluate whether the TRP2 protein is denatured depending on various egg lecithin concentrations and storage temperatures, the dispersion protein (TRP2) and the TRP2 treated in cells (TRP2 transduction) were confirmed.

Based on the results thereof, as shown in FIG. 11B, it was found that the TRP2 protein was not denatured at all at various storage temperatures and egg lecithin concentrations.

### Example 8: Confirmation of in-vivo effect of TRP2 formulation

A change in the therapeutic effect of the TRP formulation including a dispersant (Cremophor, egg lecithin or soybean lecithin) was confirmed when the same was administered to an animal model. In the above examples, effects of increasing the dispersibility of TRP2 protein including the phospholipid such as egg lecithin, Cremophor, soybean lecithin, glyceryl trioleate, and LysoPC, improving safety, enhancing the drug efficacy due to an increase in cell permeation efficiency, and improving stability were confirmed. A peritoneal fibrosis model was used to confirm enhancement of the therapeutic effect based on these effects.

For animal experiments, a peritoneal dialysis catheter (Access technologies, Rat-O-Port, 7fr x 24" silicone catheter with round tip) was inserted into the abdominal cavity of male Sprague-Dawley rats weighing 250-280 g. Every day from one week after surgery, the control group was administered with 20 ml of a saline, and the experimental group was administered with a peritoneal dialysis fluid including 4.25% high-concentration glucose. In order to confirm the effect of the TRP2 formulation on treating peritoneal fibrosis, various doses of TRP2 were mixed with the peritoneal dialysis fluid and injected. As such, the TRP2 protein drug was administered once a week. Four weeks after administration of the peritoneal dialysis fluid, each animal was sacrificed, and the peritoneum was collected, fixed in 10% neutral formalin, embedded in paraffin, and stained with Masson's Trichrome, and thus the extent of histological fibrosis was determined by measuring the fibrosis thickness of at least 30 sites.

In the case of conventional undispersed TRP2, as shown in Table 5 and in FIG. 12, the peritoneum in the normal control (con) had a thickness of about 32 µm, but when the high-concentration peritoneal dialysis fluid (PDF) was administered for 4 weeks, the thickness of the peritoneum was determined to be 230 µm or more due to peritoneal fibrosis. In this procedure, when the conventional TRP2 was mixed and administered, fibrosis was reduced by about 1/2 at a dose of 500 µg/kg.

In order to confirm the therapeutic effect using a dispersant such as Cremophor, egg lecithin, or soybean lecithin, TRP2 was dispersed using 10% Cremophor, 0.1% egg lecithin, and 0.1% soybean lecithin, and various doses thereof were mixed with the peritoneal dialysis fluid and administered once a week.

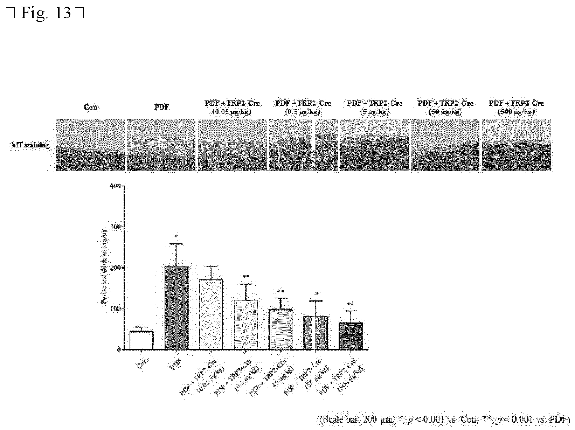

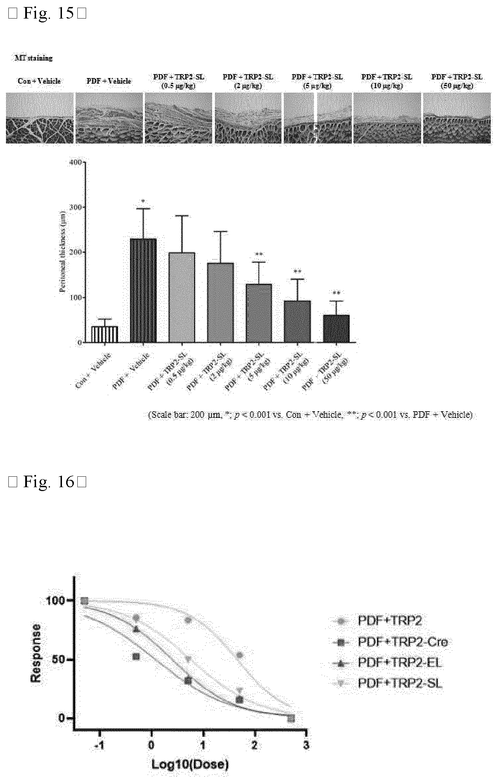

Based on the results thereof, as shown in Tables 6 to 8 below and in FIGS. 13 to 15, when the dispersant was used, the peritoneal fibrosis inhibitory effect of TRP2 was greatly increased. For the therapeutic effects of TRP2 using these dispersants, as shown in Table 9 below and in FIG. 16, when Cremophor, egg lecithin and soybean lecithin were used as the dispersant, with regard to the 50% effective dose (effective dose 50, ED50), the therapeutic effect was increased about 37 times, about 18 times, and about 8 times, respectively.

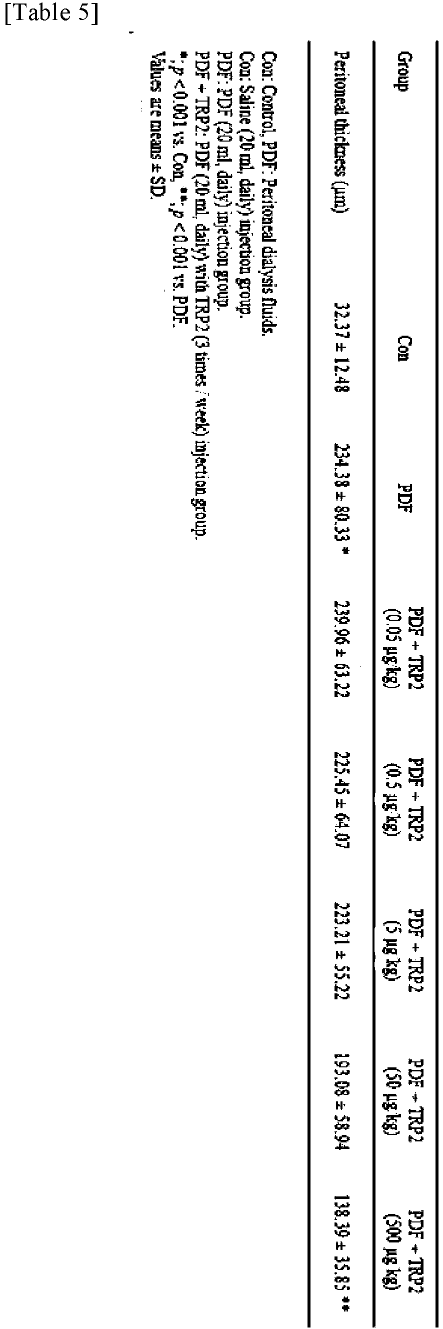

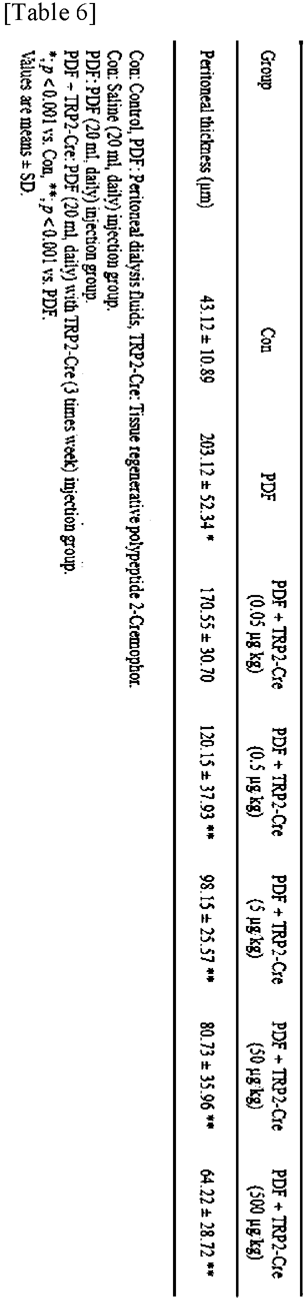

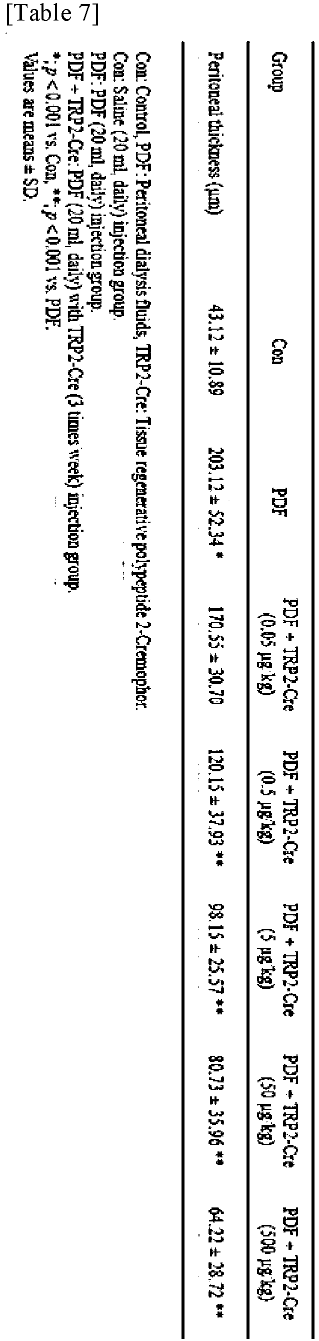

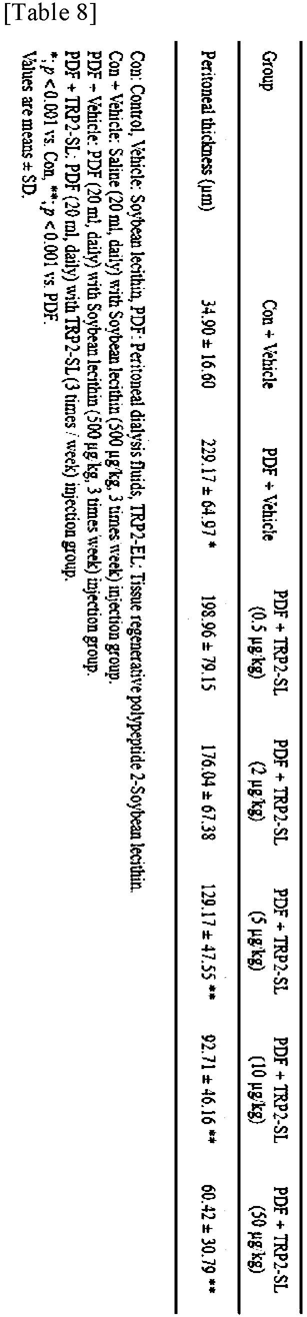

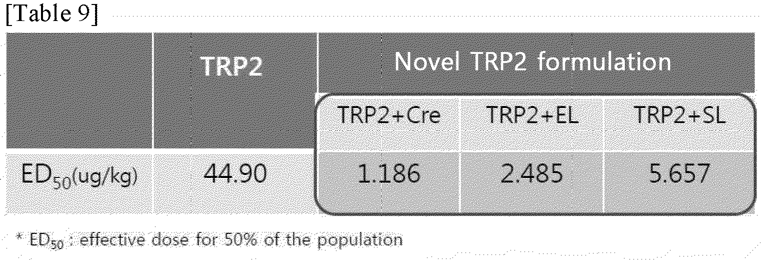

### [Industrial Applicability]

When the pharmaceutical formulation according to the present invention is used, dispersibility is improved through inhibition of aggregation of non-activated TRP, cell permeability is increased, cytotoxicity is reduced, stability of protein medicines is increased, and therapeutic effects are also improved.

Although specific embodiments of the present invention have been disclosed in detail as described above, it will be obvious to those of ordinary skill in the art that the description is merely of preferable exemplary embodiments, and is not to be construed as limiting the scope of the present invention. Therefore, the scope of the present invention will be defined by the appended claims.

### [Sequence List Free Text]

An electronic file is attached.

## Claims

1. A pharmaceutical formulation, comprising;
(a) a non-activated polypeptide TRP(tissue regenerative polypeptide) containing a PTD (protein transduction domain), which enables cell membrane permeation without aid of a cell membrane receptor, an FAD (furin activation domain), having at least one proprotein convertase cleavage site that can be cleaved with a proprotein convertase to activate a non-activated TRD (tissue regeneration domain) in a cell, and a TRD, which is activated through cleavage with the proprotein convertase of FAD to promote tissue growth or formation in a cell or induce tissue regeneration; and
(b) a dispersant selected from the group consisting of lecithin, Cremophor(CAS number 61791-12-6) and glyceryl trioleate,
wherein in the non-activated polypeptide TRP(tissue regenerative polypeptide) is TRP2(tissue regenerative polypeptide 2) represented by the amino acid sequence of SEQ ID NO:28.

2. The pharmaceutical formulation according to claim 1, wherein the lecithin is selected from the group consisting of egg lecithin, soybean lecithin, sunflower oil lecithin, canola lecithin, cottonseed lecithin, and animal fat derived lecithin.

3. The pharmaceutical formulation according to claim 1, wherein the proprotein convertase is furin.

4. The pharmaceutical formulation according to claim 1, for use in oral administration or injection.

5. The pharmaceutical formulation according to claim 1, wherein the dispersant is contained in an amount of 100 to 50,000 parts by weight based on 100 parts by weight of the non-activated polypeptide TRP.

6. The pharmaceutical formulation according to claim 5, for use in the treatment of fibrotic disease.

7. The pharmaceutical formulation for use according to claim 6, wherein the lecithin is egg lecithin or soybean lecithin.

## Patentansprüche

1. Pharmazeutische Formulierung, die Folgendes umfasst:
(a) ein nicht aktiviertes Polypeptid TRP (Geweberegenerationspolypeptid), das eine PTD (Proteintransduktionsdomäne), die eine Zellmembranpermeation ohne Hilfe eines Zellmembranrezeptor ermöglicht, eine FAD (Furinaktivierungsdomäne), die zumindest eine Proprotein-Convertase-Spaltstelle aufweist, die mit einer Proprotein-Convertase gespalten werden kann, um eine nicht aktivierte TRD (Geweberegenerationsdomäne) in einer Zelle zu aktivieren, und eine TRD enthält, die durch Spaltung mit der Proprotein-Convertase von FAD aktiviert wird, um Gewebewachstum oder -bildung in einer Zelle zu fördern oder Geweberegeneration zu induzieren; und
(b) ein Dispergiermittel, das aus der aus Lecithin, Cremophor (CAS Nr. 61791-12-6) und Glyceryltrioleat bestehenden Gruppe ausgewählt ist,
wobei das nicht aktivierte Polypeptid TRP (Geweberegenerationspolypeptid) TRP2 (Geweberegenerationspolypeptid 2) ist, das durch die Aminosäuresequenz der SEQ ID NO: 28 dargestellt ist.

2. Pharmazeutische Formulierung nach Anspruch 1, wobei das Lecithin aus der aus Eilecithin, Sojabohnenlecithin, Sonnenblumenöllecithin, Rapslecithin, Baumwollsamenlecithin und aus tierischem Fett stammendem Lecithin bestehenden Gruppe ausgewählt ist.

3. Pharmazeutische Formulierung nach Anspruch 1, wobei die Proprotein-Convertase Furin ist.

4. Pharmazeutische Formulierung nach Anspruch 1 zur Verwendung durch orale Verabreichung oder Injektion.

5. Pharmazeutische Formulierung nach Anspruch 1, wobei das Dispergiermittel in einer Menge von 100 bis 50.000 Gewichtsteilen bezogen auf 100 Gewichtsteile des nicht aktivierten Polypeptids TRP enthalten ist.

6. Pharmazeutische Formulierung nach Anspruch 5 zur Verwendung bei der Behandlung einer fibrotischen Erkrankung.

7. Pharmazeutische Formulierung zur Verwendung nach Anspruch 6, wobei das Lecithin Eilecithin oder Sojabohnenlecithin ist.

## Revendications

1. Formulation pharmaceutique, comprenant :
(a) un polypeptide TRP (polypeptide de régénération tissulaire) non activé contenant un PTD (domaine de transduction de protéine), qui permet une perméation de membrane cellulaire sans l'aide d'un récepteur de membrane cellulaire, un FAD (domaine d'activation de furine), présentant au moins un site de clivage de proprotéine convertase qui peut être clivé avec une proprotéine convertase pour activer un TRD (domaine de régénération tissulaire) non activé dans une cellule, et un TRD, qui est activé par clivage avec la proprotéine convertase de FAD pour favoriser la croissance ou la formation de tissu dans une cellule ou induire la régénération tissulaire ; et
(b) un dispersant choisi dans le groupe constitué de la lécithine, du Crémophor (numéro CAS 61791-12-6) et du trioléate de glycéryle,
dans laquelle dans le polypeptide TRP (polypeptide régénératif tissulaire) non activé se trouve du TRP2 (polypeptide de régénération tissulaire 2) représenté par la séquence d'acides aminés de SEQ ID NO :28.

2. Formulation pharmaceutique selon la revendication 1, dans laquelle la lécithine est choisie dans le groupe comprenant la lécithine d'œuf, la lécithine de soja, la lécithine d'huile de tournesol, la lécithine de canola, la lécithine de graine de coton et la lécithine dérivée de graisse animale.

3. Formulation pharmaceutique selon la revendication 1, dans laquelle la proprotéine convertase est la furine.

4. Formulation pharmaceutique selon la revendication 1, pour utilisation en administration orale ou en injection.

5. Formulation pharmaceutique selon la revendication 1, dans laquelle le dispersant est contenu en une quantité de 100 à 50 000 parties en poids sur la base de 100 parties en poids du polypeptide TRP non activé.

6. Formulation pharmaceutique selon la revendication 5, pour utilisation dans le traitement d'une maladie fibrotique.

7. Formulation pharmaceutique pour utilisation selon la revendication 6, dans laquelle la lécithine est de la lécithine d'œuf ou de la lécithine de soja.
